(19) Europäisches Patentamt
European Patent Office
Office européen des brevets

(11) **EP 2 363 166 A1**

(12) **EUROPEAN PATENT APPLICATION**

(43) Date of publication:
**07.09.2011 Bulletin 2011/36**

(51) Int Cl.:
*A61N 1/368* (2006.01)      *A61N 1/372* (2006.01)

(21) Application number: **10250952.8**

(22) Date of filing: **19.05.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **20.05.2009 US 469564**

(71) Applicant: **PACESETTER, INC.
Sylmar, CA 91392-9221 (US)**

(72) Inventors:
• **Bharmi, Rupinder
  Canyon Country, CA 91387 (US)**
• **Bornzin, Gene A.
  Simi Valley, CA 93065 (US)**

(74) Representative: **Phillips, Emily Elizabeth et al
  Kilburn & Strode LLP
  20 Red Lion Street
  London WC1R 4PJ (GB)**

(54) **Electrolyte monitoring using implanted cardiac rhythm management device**

(57)     A system for diagnosing an electrolyte level of a patient, comprising means for recording intra-cardiac electrograms from a plurality of sites; and means for quantifying the electrolyte level based upon a comparative analysis of the recorded intra-cardiac electrograms from at least two of the sites.

ELECTROLYTE LEVEL MONITORING

FIG. 3

**Description**

FIELD OF THE DISCLOSURE

The present disclosure generally relates to medical devices. More specifically, the present disclosure relates to cardiac rhythm management devices that monitor electrolyte levels.

BACKGROUND

A number of patients with heart failure have renal insufficiency, which leads to hyperkalemia. Renal insufficiency is amongst the strongest predictors of mortality in patients with heart failure. Greater than 90% of patients with heart failure are on drugs including ACE inhibitors, angiotensin II receptor blockers (ARB), Beta-blockers, loop diuretics, digoxin and potassium supplements. Optimal medical therapy of patients with symptomatic heart failure requires simultaneous utilization of multiple neurohormonal antagonists that alone and especially in combination increase the risk of hyperkalemia.

Patients with renal dysfunction are at higher risk of developing hyperkalemia and require vigilant monitoring. Joint American College of Cardiology, American Heart Association (ACC/AHA) guidelines for minimizing risk of hyperkalemia in heart failure patients treated with aldosterone antagonists recommend a close monitoring of serum potassium and renal function: to be checked in three days, at one week after initiation of therapy; and at least monthly for the first three months.

Presently, blood test monitoring is used, which can be inconvenient and uncomfortable. It has been reported that 30-60% of patients do not receive recommended surveillance. Because these patients form a high percentage of the cardiac rhythm management device population, it would be desirable to provide a cardiac rhythm management device based detection of electrolyte imbalances, such as potassium abnormalities.

Moreover, it has been shown that hypokalemia can result in electrical storms. As a result, proper cardiac rhythm management does not occur even during shock therapy, unless the underlying condition is corrected. In order to better manage these patients, it would be desirable to provide a cardiac rhythm management device based detection of electrolyte imbalances.

SUMMARY

According to an aspect of the present disclosure, a method is provided for diagnosing an electrolyte level with a cardiac rhythm management device. The method includes recording intra-cardiac electrograms from multiple sites. The electrolyte level is determined based upon a comparative analysis of the intra-cardiac electrograms from at least two of the sites.

In another embodiment, a medical device diagnoses an electrolyte level of a patient. The medical device has at least a first electrode and a second electrode. The first electrode is implanted at a first cardiac site to facilitate generating a first intra-cardiac electrogram. The second electrode is implanted at a second cardiac site to facilitate generating a second intra-cardiac electrogram. The medical device also has a processing system that analyzes at least one feature from the first and second intra-cardiac electrograms to quantify the electrolyte level. This is done by comparing different sites and different time segments between the different sites of recording.

In yet another embodiment, a system diagnoses an electrolyte level of a patient. The system includes means for recording intra-cardiac electrograms from multiple sites. The system also includes means for quantifying the electrolyte level based upon a comparative analysis of the stored intra-cardiac electrograms from at least two of the sites.

The foregoing has outlined rather broadly the features and technical advantages of the present disclosure in order that the detailed description of embodiments of the disclosure that follows may be better understood. Additional features and advantages of the disclosure will be described hereinafter which form the subject of the claims of the disclosure. It should be appreciated by those skilled in the art that the conception and specific embodiments disclosed may be readily utilized as a basis for modifying or designing other structures for carrying out the same purposes of the present disclosure. It should also be realized by those skilled in the art that such equivalent constructions do not depart from the spirit and scope of the disclosure as set forth in the appended claims. The novel features which are believed to be characteristic of the disclosure, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present disclosure.

BRIEF DESCRIPTION OF THE DRAWINGS

For a more complete understanding of the present disclosure, reference is now made to the following descriptions taken in conjunction with the accompanying drawings.

FIGURE 1 is a simplified drawing illustrating an implantable cardiac rhythm management device with leads implanted in the heart of a patient.

FIGURE 2 is a functional block diagram of the implantable cardiac rhythm management device of FIGURE 1, illustrating exemplary elements including components for monitoring electrolyte levels.

FIGURE 3 is a flow chart illustrating an exemplary electrolyte monitoring process.

FIGURE 3A is a flow chart illustrating an exemplary electrolyte level calculation sub-process.

FIGURE 4 is a flow chart illustrating an exemplary general calibration process.

FIGURE 5 is a flow chart illustrating an exemplary patient specific calibration process.

DETAILED DESCRIPTION

The following description includes the best mode presently contemplated for practicing the invention. The description is not to be taken in a limiting sense but is merely for the purpose of describing the general principles of the illustrative embodiments. The scope of the invention should be ascertained with reference to the claims. In the description that follows, like numerals or reference designators will refer to like parts or elements throughout.

Overview of Implantable Device

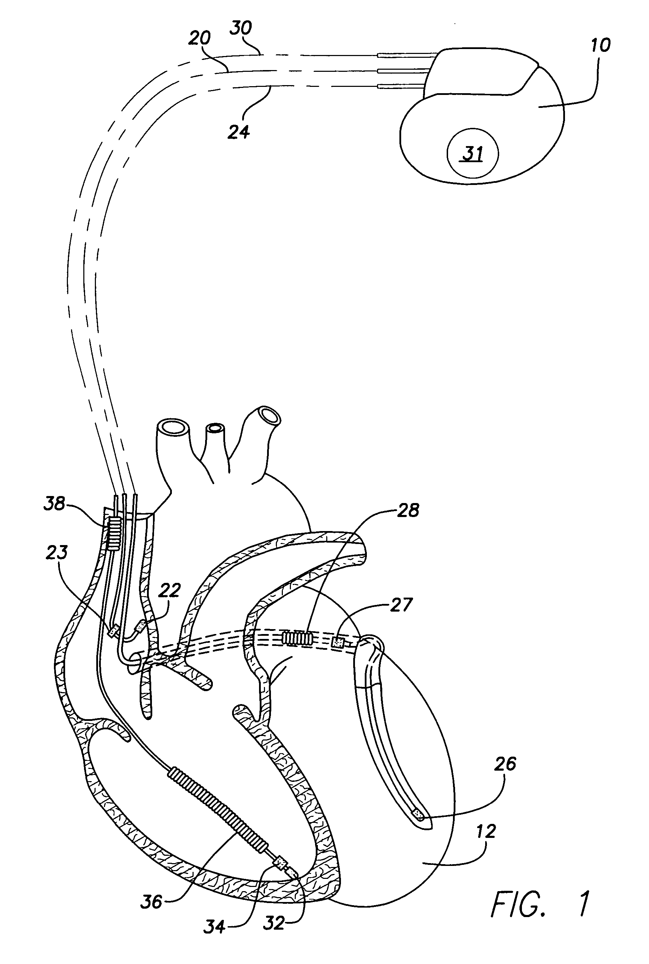

With reference to FIG. 1, there is a stimulation device 10 in electrical communication with the heart 12 of a patient by way of three leads, 20, 24 and 30, suitable for delivering multi-chamber stimulation and shock therapy. To sense atrial cardiac signals and to provide right atrial chamber stimulation therapy, the stimulation device 10 is coupled to an implantable right atrial lead 20 having at least an atrial tip electrode 22, which typically is implanted in the right atrial appendage, and an atrial ring electrode 23.

To sense left atrial and ventricular cardiac signals and to provide left chamber pacing therapy, the stimulation device 10 is coupled to a "coronary sinus" lead 24 designed for placement in the "coronary sinus region" via the coronary sinus or for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus. Accordingly, an exemplary coronary sinus lead 24 is designed to receive atrial and ventricular cardiac signals and to deliver left ventricular pacing therapy using at least a left ventricular tip electrode 26, left atrial pacing therapy using at least a left atrial ring electrode 27, and shocking therapy using at least a left atrial coil electrode 28.

The stimulation device 10 is also shown in electrical communication with the heart by way of an implantable right ventricular lead 30 having, in this embodiment, a right ventricular tip electrode 32, a right ventricular ring electrode 34, a right ventricular (RV) coil electrode 36, and a superior vena cava (SVC) coil electrode 38. Typically, the right ventricular lead 30 is transvenously inserted into the heart so as to place the right ventricular tip electrode 32 in the right ventricular apex so the RV coil electrode 36 is positioned in the right ventricle and the SVC coil electrode 38 is positioned in the superior vena cava. Accordingly, the right ventricular lead 30 is capable of receiving cardiac signals, and delivering stimulation in the form of pacing and shock therapy to the right ventricle. To provide a "vibratory alert" signal (from a motor with an offset mass that can be provided in the device can), an additional electrode 31 can be provided in proximity to the device can.

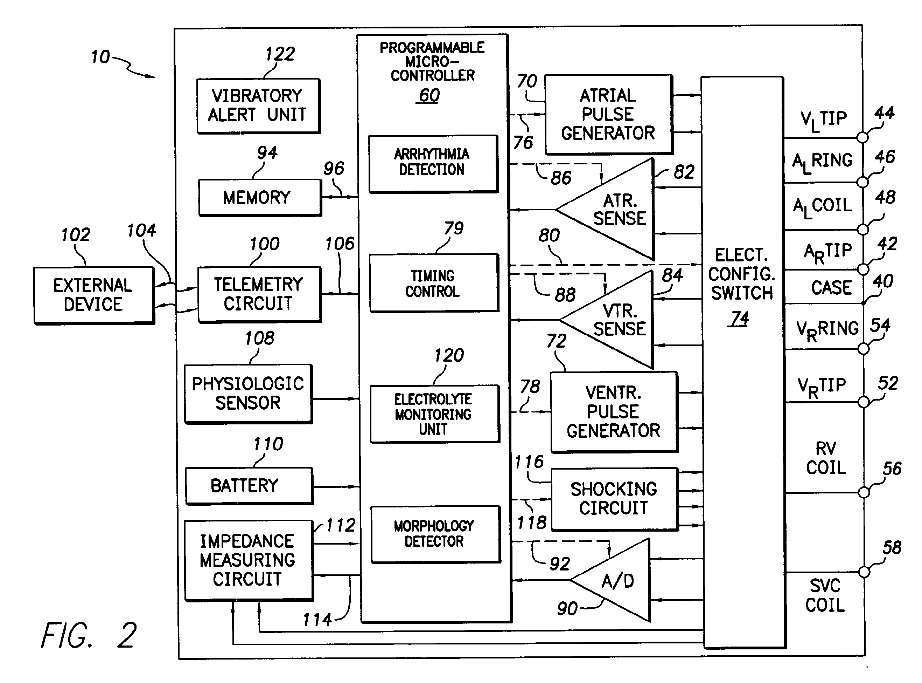

As illustrated in FIG. 2, a simplified block diagram is shown of the multi-chamber implantable stimulation device 10, which is capable of treating both fast and slow arrhythmias with stimulation therapy, including cardioversion, defibrillation, and pacing stimulation. While a particular multi-chamber device is shown, this is for illustration purposes only, and one of skill in the art could readily duplicate, eliminate or disable the appropriate circuitry in any desired combination to provide a device capable of treating the appropriate chamber(s) with cardioversion, defibrillation and pacing stimulation.

The housing 40 for the stimulation device 10, shown schematically in FIG. 2, is often referred to as the "can", "case" or "case electrode" and may be programmably selected to act as the return electrode for all "unipolar" modes. The housing 40 may further be used as a return electrode alone or in combination with one or more of the coil electrodes, 28, 36 and 38, for shocking purposes. The housing 40 further includes a connector (not shown) having a plurality of terminals, 42, 44, 46, 48, 52, 54, 56 and 58 (shown schematically and, for convenience, the names of the electrodes to which they are connected are shown next to the terminals).

As such, to achieve right atrial sensing and pacing, the connector includes at least a right atrial tip terminal ($A_R$ TIP) 42 adapted for connection to the atrial tip electrode 22 and a right atrial ring ($A_R$ RING) electrode (not shown) adapted for connection to the right atrial ring electrode 23. To achieve left chamber sensing, pacing and shocking, the connector includes at least a left ventricular tip terminal ($V_L$ TIP) 44, a left atrial ring terminal ($A_L$ RING) 46, and a left atrial shocking terminal ($A_L$ COIL) 48, which are adapted for connection to the left ventricular ring electrode 26, the left atrial tip electrode 27, and the left atrial coil electrode 28, respectively. To support right chamber sensing, pacing and shocking, the connector further includes a right ventricular tip terminal ($V_R$ TIP) 52, a right ventricular ring terminal ($V_R$ RING) 54, a right ventricular shocking terminal (RV COIL) 56, and an SVC shocking terminal (SVC COIL) 58, which are adapted for connection to the right ventricular tip electrode 32, right ventricular ring electrode 34, the RV coil electrode 36, and the SVC coil electrode 38, respectively. To provide the "vibratory alert" signal, a vibratory alert unit 122 generates a signal for an additional terminal (not shown) for connection to the vibratory alert electrode 31. In one embodiment, the vibratory alert will alert the patient, and then a home monitor can be used to transfer the information associated with the alert from the device 10 to an attending medical professional, who can take the appropriate clinical action.

At the core of the stimulation device 10 is a programmable microcontroller 60, which controls the various modes of stimulation therapy. As is well known in the art, the microcontroller 60 (also referred to as a control unit) typically includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 60 includes the ability to process or monitor input signals (data) as controlled by program code stored in a designated block of memory. The details of the design and operation of the microcontroller 60 are not critical to the invention. Rather, any suitable microcontroller 60 may be used that carries out the functions described. The use of microprocessor-based control circuits for performing timing and data analysis functions are well known in the art.

As shown in FIG. 2, an atrial pulse generator 70 and a ventricular pulse generator 72 generate pacing stimulation pulses for delivery by the right atrial lead 20, the right ventricular lead 30, and/or the coronary sinus lead 24 via an electrode configuration switch 74. It is understood that in order to provide stimulation therapy in each of the four chambers of the heart, the atrial and ventricular pulse generators, 70 and 72, may include dedicated, independent pulse generators, multiplexed pulse generators or shared pulse generators. The pulse generators, 70 and 72, are controlled by the microcontroller 60 via appropriate control signals, 76 and 78, respectively, to trigger or inhibit the stimulation pulses.

The microcontroller 60 further includes timing control circuitry 79 that controls the timing of such stimulation pulses (e.g., pacing rate, atrioventricular (AV) delay, atrial interconduction (A-A) delay, or ventricular interconduction (V-V) delay, etc.) as well as to keep track of the timing of refractory periods, blanking intervals, noise detection windows, evoked response windows, alert intervals, marker channel timing, etc., as is well known in the art. The switch 74 includes multiple switches for connecting the desired electrodes to the appropriate I/O circuits, thereby providing complete electrode programmability. Accordingly, the switch 74, in response to a control signal 80 from the microcontroller 60, determines the polarity of the stimulation pulses (e.g., unipolar, bipolar, combipolar, etc.) by selectively closing the appropriate combination of switches (not shown) as is known in the art.

Atrial sensing circuits 82 and ventricular sensing circuits 84 may also be selectively coupled to the right atrial lead 20, the coronary sinus lead 24, and the right ventricular lead 30, through the switch 74 for detecting the presence of cardiac activity in each of the four chambers of the heart. Accordingly, the atrial (ATR. SENSE) and ventricular (VTR. SENSE) sensing circuits, 82 and 84, may include dedicated sense amplifiers, multiplexed amplifiers or shared amplifiers and may receive control signals 86, 88 from the controller 60. The switch 74 determines the "sensing polarity" of the cardiac signal by selectively closing the appropriate switches, as is also known in the art. In this way, the clinician may program the sensing polarity independent of the stimulation polarity. Each sensing circuit, 82 and 84, preferably employs one or more low power, precision amplifiers with programmable gain and/or automatic gain control, band pass filtering, and a threshold detection circuit, as known in the art, to selectively sense the cardiac signal of interest. The automatic gain control enables the device 10 to effectively address the difficult problem of sensing the low amplitude signal characteristics of atrial or ventricular fibrillation. The outputs of the atrial and ventricular sensing circuits, 82 and 84, are connected to the microcontroller 60 which, in turn, are able to trigger or inhibit the atrial and ventricular pulse generators, 70 and 72, respectively, in a demand fashion in response to the absence or presence of cardiac activity in the appropriate chambers of the heart.

For arrhythmia detection, the device 10 utilizes the atrial and ventricular sensing circuits, 82 and 84, to sense cardiac signals to determine whether a rhythm is physiologic or pathologic. As used herein "sensing" is reserved for the noting of an electrical signal, and "detection" is the processing of these sensed signals and noting the presence of an arrhythmia. The timing intervals between sensed events (e.g., P-waves, R-waves, and depolarization signals associated with fibrillation which are sometimes referred to as "F-waves" or "Fib-waves") are then classified by the microcontroller 60 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, antitachycardia pacing, cardioversion shocks or defibrillation shocks).

Cardiac signals are also applied to the inputs of an analog-to-digital (A/D) data acquisition system 90. The data acquisition system 90 is configured to acquire intra-cardiac electrogram (IEGM) signals, convert the raw analog data into a digital signal, and store the digital signals for later processing and/or telemetric transmission to an external device 102. The data acquisition system 90 is coupled to the right atrial lead 20, the coronary sinus lead 24, and the right ventricular lead 30 through the switch 74 to sample cardiac signals across any pair of desired electrodes. The controller 60 controls the data acquisition system via control signals 92.

The microcontroller 60 is further coupled to a memory 94 by a suitable data/address bus 96. The programmable operating parameters used by the microcontroller 60 are stored and modified, as required, in order to customize the operation of the stimulation device 10 to suit the needs of a particular patient. Such operating parameters define, for example, pacing pulse amplitude or magnitude, pulse duration, electrode polarity, rate, sensitivity, automatic features, arrhythmia detection criteria, and the amplitude, wave shape and vector of each shocking pulse to be delivered to the patient's heart within each respective tier of therapy. Other pacing parameters include base rate, rest rate and circadian base rate.

Advantageously, the operating parameters of the implantable device 10 may be non-invasively programmed into the memory 94 through a telemetry circuit 100 in telemetric communication with the external device 102, such as a programmer, trans-telephonic transceiver, a diagnostic system analyzer, or even a cellular telephone. The telemetry circuit 100 is activated by the microcontroller by a control signal 106. The telemetry circuit 100 advantageously allows intra-cardiac electrograms and status information relating to the operation of the device 10 (as contained in the microcontroller 60 or memory 94) to be sent to the external device 102 through an established communication link 104. In one embodiment, the stimulation device 10 further includes a physiologic sensor 108, commonly referred to as a "rate-responsive" sensor because it adjusts pacing stimulation rate according to the exercise state of the patient. However, the physiological sensor 108 may further be used to detect changes in cardiac output, changes in the physiological condition of the heart, or diurnal changes in activity (e.g., detecting sleep and wake states). Accordingly, the microcontroller 60 responds by adjusting the various pacing parameters (such as rate, AV Delay, V-V Delay, etc.) at which the atrial and ventricular pulse generators, 70 and 72, generate stimulation pulses. While shown as being included within the stimulation device 10, it is to be understood that the physiologic sensor 108 may also be external to the stimulation device 10, yet still be implanted within or carried by the patient.

The stimulation device additionally includes a battery 110, which provides operating power to all of the circuits shown in FIG. 2. For the stimulation device 10, which employs shocking therapy, the battery 110 is capable of operating at low current drains for long periods of time, and is capable of providing high-current pulses (for capacitor charging) when the patient requires a shock pulse. The battery 110 also has a predictable discharge characteristic so that elective replacement time can be detected. In one embodiment, the device 10 employs lithium/silver vanadium oxide batteries. As further shown in FIG. 2, the device 10 has an impedance measuring circuit 112 enabled by the microcontroller 60 via a control signal 114.

In the case where the stimulation device 10 is intended to operate as an implantable cardioverter/defibrillator (ICD) device, it detects the occurrence of an arrhythmia and automatically applies an appropriate electrical shock therapy to the heart aimed at terminating the detected arrhythmia. To this end, the microcontroller 60 further controls a shocking circuit 116 by way of a control signal 118. The shocking circuit 116 generates shocking pulses of low (up to 0.5 joules), moderate (0.5-10 joules), or high energy (11 to 40 joules), as controlled by the microcontroller 60. Such shocking pulses are applied to the heart 12 through at least two shocking electrodes, and as shown in this embodiment, selected from the left atrial coil electrode 28, the RV coil electrode 36, and/or the SVC coil electrode 38. As noted above, the housing 40 may function as an active electrode in combination with the RV coil electrode 36, or as part of a split electrical vector using the SVC coil electrode 38 or the left atrial coil electrode 28 (i.e., using the RV electrode as a common electrode). Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 joules), delivered asynchronously (since R-waves may be too disorganized), and pertaining exclusively to the treatment of fibrillation. Accordingly, the microcontroller 60 is capable of controlling the synchronous or asynchronous delivery of the shocking pulses.

Finally, with regard to FIG. 2, the microcontroller 60 includes a morphology detector for tracking various morphological features within electrical cardiac signals, including intervals between polarization events, elevations between polarization events, durations of polarization events and amplitudes of polarization events. The microcontroller 60 can also includes an electrolyte monitoring unit 120 for monitoring electrolyte levels, based on changes in the features tracked by the morphology detector. A warning unit (not shown) controls delivery of warning signals to the patient indicative of an electrolyte imbalance. In particular, the warning unit controls a tickle circuit that generates subcutaneous perceptible warning signals via the vibratory alert electrode 31 (FIG. 1). The device case 40 may be used as the return electrode for the vibratory alert signal. Thereafter, the warning unit controls the telemetry system 100 to transmit warning signals to an external handheld warning device 102 for confirmation. Additionally, a therapy control unit may be provided to control therapy based upon the detection of an electrolyte imbalance. The operation of these devices will be described below with reference to the remaining figures.

Electrolyte Monitoring

The remaining figures, flow charts, graphs and other diagrams illustrate the operation and novel features of the stimulation device 10 as configured in accordance with exemplary embodiments of the invention. In the flow charts, the various algorithmic steps are summarized in individual "blocks". Such blocks describe specific actions or decisions made or carried out as the algorithm proceeds. Where a microcontroller (or equivalent) is employed, the flow charts provide the basis for a "monitoring/detection program" that may be used by such a microcontroller (or equivalent) to monitor for electrolyte imbalance. Those skilled in the art may readily write such a program based on the flow charts and other descriptions presented herein.

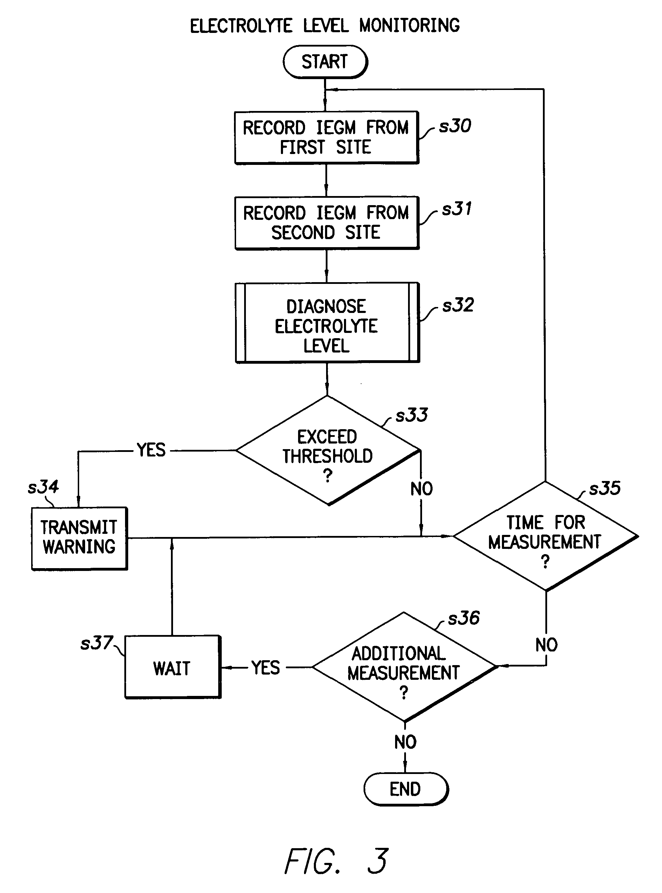

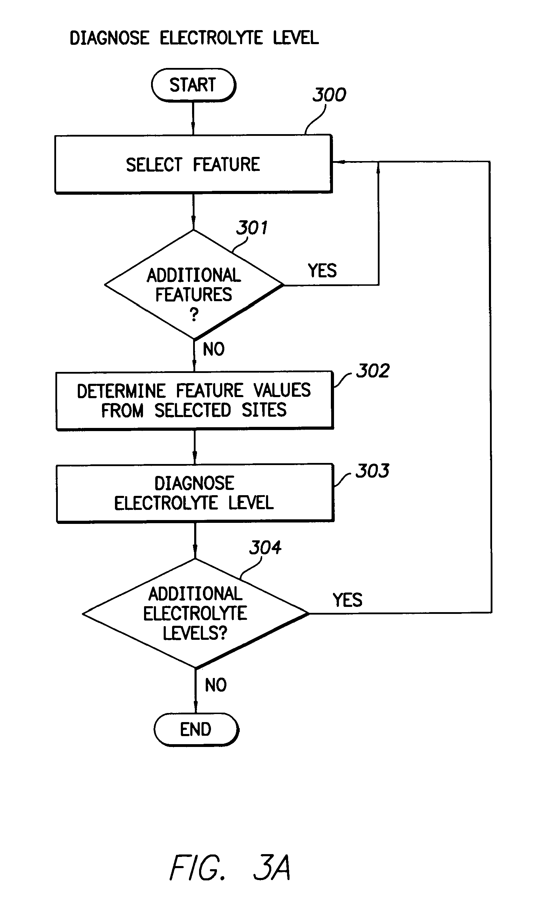

Referring now to FIG. 3, a process for electrolyte level monitoring will be discussed. According to an aspect of the present invention, a comparative analysis of the electrolyte level is based upon locations of recordings. Initially, at block S30 an intra-cardiac electrogram is recorded from a first site by a cardiac rhythm management device, such as the implanted device 10 described above. In one embodiment, the recordings originate from the right atrium, the right ventricle, the left ventricle, the Purkinje system, the AV node, or the papillary muscles. More specific examples of these recording locations include measuring from the can 40 to the atrial tip electrode 22, measuring from the right ventricle tip electrode 32 to the can 40, measuring from the right ventricle tip electrode 32 to the right ventricular ring electrode 34; and measuring from the SVC coil electrode 38 to the can 40. Of course other measurements sites are contemplated, with the foregoing list merely be exemplary.

At block S31 an intra-cardiac electrogram is recorded from an additional site, which is different from the first site. Different exemplary regions for the recording include the atrium, the AV node, Purkinje fibers, ventricles, and the papillary muscles. Each region has different types of action potentials that reflect in the intra-cardiac electrograms. Thus, the specificity of the measurements is enhanced by recording from the additional site. By monitoring the different regions of the action potential from different sites, a comparative analysis of the electrolyte changes based upon these different recordings can be developed. That is, at block S32 the electrolyte level is calculated based upon the intra-cardiac electrograms recorded from the different sites. Although the description refers to intra-cardiac electrograms from only two locations, the present invention contemplates calculations based on recordings from more than two sites. More detail of the electrolyte level calculation will be provided below with respect to FIG. 3A.

After the electrolyte level has been calculated, at block S33 it is determined whether a threshold electrolyte level has been exceeded. If the threshold is exceeded, at block S34 a warning is transmitted, for example, to the external device 102. In one embodiment, the warning includes transmitting the diagnosed electrolyte level to the patient, for example from the external device 102 to a cellular phone of the patient, e.g., via an instant message or phone call. In another embodiment, a database receives the warning transmitted from the external device 102. A physician can then be notified. In this embodiment, the physician could request the patient to come into the office because the patient is showing signs of an electrolyte imbalance. Consequently, the physician could attempt to correct the imbalance before it becomes malignant.

If the threshold was not exceeded or after the warning has been transmitted, at block S35 it is determined whether it is time to record the next intra-cardiac electrograms. If so, the logic returns to block S30. If it is not time, at block S36 it is determined whether additional recordings are even desired. If so, at block S37 the system waits until it is time for the next recording and the logic returns to block S35. If no additional recordings are desired, the processing ends. Thus, it can be seen that a cardiac rhythm management device can monitor a patient's electrolyte levels based upon a comparative analysis, and then notify when an electrolyte imbalance has been detected.

Calculating an electrolyte level will now be described in more detail with respect to FIG. 3A. Different action potentials regions correlate to different electrolytes, and accordingly different regions of an intra-cardiac electrogram correlate to the different electrolytes. Thus, by selecting the appropriate action potentials regions (or features derived from those action potentials), an electrolyte status can be diagnosed. At block 300 a feature is identified, based upon the electrolyte to be diagnosed. For example, potassium can be recognized from analysis of repolarization features. The depolarization features and the QRS features also change with potassium, so some of these features might also be evaluated when diagnosing potassium levels. Depolarization features may also be analyzed to indicate sodium changes. Finally, the plateau region, i.e., ST area, indicates calcium levels.

A non-limiting list of specific features that can be analyzed includes an amplitude, a slew rate, an integral, and a mean integral of relevant regions of the recorded intra-cardiac electrograms. For example, to diagnose a potassium level an integral of a T wave as determined from a right ventricle recording may be analyzed as well as an integral of a T wave determined from a papillary muscle recording. Multiple features can be calculated at each instance, but a combination of features will be specific to each electrolyte.

In block 301, it is determined whether additional features will also be examined. If additional features are to be considered, the logic returns to block 300 until all features have been identified. If additional features are selected, then multiple features from each of the multiple regions would be considered in the analysis. In one embodiment, changes over time are also considered. Thus, the additional selected feature could be the same feature but measured at a different time. For example, the delta change and directional change of T wave amplitudes from today's recordings could be compared to T wave amplitudes from yesterday's recordings to diagnose the electrolyte level. In order to differentiate these changes of electrolytes from cardiac ischemia, the rate of change of feature can be used, as cardiac ischemia changes are sudden, and electrolyte changes occur over days for a chronic exacerbation. For an acute exacerbation of electrolyte imbalance, the rate of change of the features may be quicker, but the specific features used for electrolyte detection are different from features associated with cardiac ischemia related changes, such as QTmax-QTend, and the ST segment.

After all the features have been identified, at block 302 feature values are determined from selected sites. One example of a feature extraction is calculating a maximum excursion relative to a QRS complex. Another feature extraction calculates the integral of the T wave. Yet another feature extraction integrates the T wave and computes the average value by dividing by time.

After the feature values have been determined, at block 303 electrolyte levels are calculated based upon the feature values. In one embodiment, electrolyte imbalances are generally identified. In another embodiment, electrolyte levels are quantified.

An example of generally identifying an electrolyte imbalance is now provided with respect to hyperkalemia and hypokalemia. Hypokalemia is generally identified by depressed T waves, broad T waves, and a T wave inversion. Other features that may indicate hypokalemia are a giant U wave larger than T waves, a sagging ST segment, a prolonged PR interval, an increased P-wave amplitude, a lengthened conduction time in the AV node, and a reduced sinus rate. Hyperkalemia may be identified as tall T waves, symmetrical T waves with a narrow base, a wide PR interval, wide P-waves with decreased amplitude and decreased slew rate, a widened QRS complex with a decreased amplitude and a decreased slew rate, and a lengthened AV conduction. Other features may further be identified to help diagnose hypokalemia and hyperkalemia. Similarly, sodium levels can be determined based upon depolarization features amongst the recordings from the different sites. Moreover, plateau features of the intra-cardiac electrograms at the different sites can be analyzed to determine calcium levels.

If electrolyte levels are quantified instead of generally diagnosed, the appropriate features values are input to a model to obtain the quantified electrolyte level. An exemplary model is shown below:

$$E = \sum_{n=1}^{k} Mn * Fn + b \qquad \text{(eq. 1)}$$

where E is the quantified electrolyte level;
M is a weighting factor;
n is the feature number;
F is the extracted feature value;
k is the total number of features; and
b is a constant.

For example, to find a potassium level, E, with k=2 features, a recorded T wave amplitude (measured from the papillary muscles) times its weighting coefficient ($M_1$) is added to a T wave amplitude (measured from the left ventricle) times its weighting coefficient ($M_2$). The sum is then added to a constant, b, to obtain the diagnosed potassium level. The potassium level can be recalculated as the measured T wave amplitudes vary. Derivation of the weighting factors will be described below in more detail. It is noted that the equation above is only a single example of a model that could be used to correlate features with specific electrolyte levels. Other models could be used, for example a fuzzy logic comparative system or a rules based system, such as: if a QRS interval wider than a threshold exists AND a T wave taller than a threshold exists, then the plasma $K^+$ is 5.5 mEq/L. In another example, a T wave above a threshold value without a P wave wider than a threshold value is ignored.

After the electrolyte level has been diagnosed, at block 304 it is determined whether any other electrolyte level diagnoses are desired. For example, if a potassium level has just been calculated, it may now be desired to calculate a sodium level. If so, the processing returns to block 300. If no further electrolyte levels are desired, the process ends and the logic returns to block S33 of FIG. 3.

In order to quantify the electrolyte levels, the electrolyte monitoring process should be calibrated. Three levels of calibration can occur to facilitate accurate quantifying of an electrolyte level based upon an analysis of the intra-cardiac electrograms. A general calibration, a patient specific single point calibration and a patient specific slope calibration are the three different calibration levels.

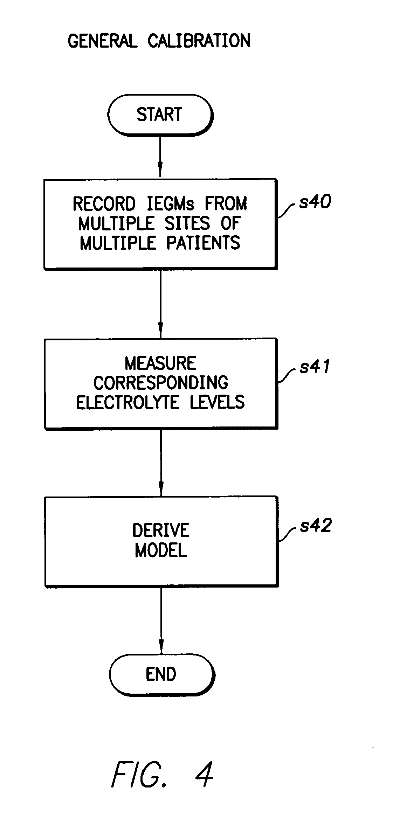

Referring now to FIG. 4, an exemplary general calibration process will be described. In one embodiment, the general calibration begins on animals, and then continues on to a human population. Initially, at block S40 intra-cardiac electrograms are recorded from multiple sites of multiple patients. At block S41 an electrolyte level is measured. For example, a physician may draw blood from a patient in order to obtain an accurate electrolyte measurement. The time when the actual electrolyte measurement occurs should correspond to when the intra-cardiac electrograms were recorded.

Once the actual electrolyte levels are known and the intra-cardiac electrograms have been recorded, a model is derived to correlate the measured electrolyte levels with the recorded intra-cardiac electrograms at block S42. In one embodiment, multiple linear regression is used to develop the model.

The equation y=mx+b illustrates a simplified model, in which the weighting factors m and b are derived based upon multiple linear regression. If y = the measured electrolyte level, and x = a feature value, for example, a T wave depression of .25 millivolts, the weighting factors m and b can be calculated. As the number of known values for y and x increase (based on additional measurements and recordings), the weighting factors are updated to increase accuracy of the model.

In one embodiment, the weighting factors are derived based upon normalized features, e.g., a percent change. One example of a feature normalization is a T wave amplitude normalized relative to a QRS complex. Thus, if a QRS complex has a value of 10 mV and the T wave has an amplitude of 2 mV, then the normalized T wave amplitude is 20%. In other embodiments, absolute values are used, instead of normalized values.

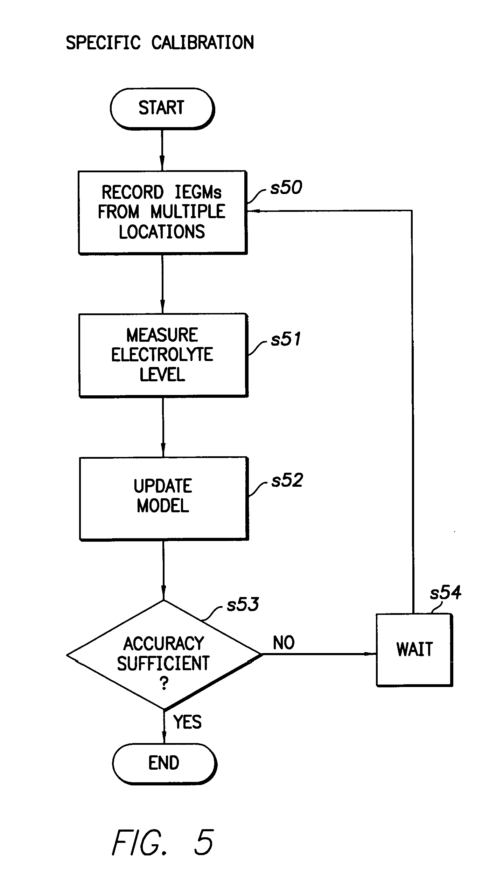

Once a cardiac rhythm management device is assigned to a specific patient, the electrolyte monitoring process can be customized to that patient to yield more precise calculations. With respect to FIG. 5, an exemplary patient specific calibration is explained. The first level of patient specific calibration is based on a single point in time, such as when the device is initially implanted or otherwise associated with the patient, or when medication to treat an electrolyte imbalance begins, or at some other time when blood would typically be drawn. The second level of patient specific calibration is based upon multiple measurements from the patient.

At block S50, intra-cardiac electrograms are recorded from multiple locations of the patient. At block S51, the patient's electrolyte level is actually measured (for example, with a blood test) at a time corresponding to when intra-cardiac electrograms are recorded.

At block S52, the general model, (described above with respect to FIG. 4) is updated based upon the measured electrolyte level and the recorded intra-cardiac electrograms. In this level of patient specific calibration, equation 1 can adopt the $M_n$ values from the general model. The constant value, b, may be set based upon the actual measured electrolyte level and the recorded intra-cardiac electrograms. For example, assume the general model has a 5.0 electrolyte level corresponding to a feature value, $F_1$, = 10, a derived weighting factor $M_1$ of .5 and b calculated to be 0. If the individual patient had a 6.0 measured electrolyte level with the same feature value $F_1$, = 10, the individual patient's b would be set to 1 in the single point calibration process of block S52.

After the single point calibration has been completed, at block S53 it is determined whether the accuracy is sufficient. If so, the process ends. If not, multiple data points will be associated with a patient. At block S54, a specified time period is allowed to pass. In one embodiment, the measurements occur at the same time of the day, thus some multiple of 24 hours is allowed to elapse before the process returns to block S50.

If additional measurements and recordings occur, the model is updated based upon the additional data to further refine the model to become more accurate for the specific patient. Multiple linear regression may be used to customize the model with refined weighting factors, i.e., both weighting factor, $M_n$ and b, values can be updated. The more data provided from the specific patient, the more accurate the model will become. In another embodiment, the additional data can refine the model based upon a minimization of a least squared error calculation.

The patient specific customization is performed by a programmer in one embodiment. In another embodiment, the calculations occur in the device itself, such as in the electrolyte monitoring unit 120.

Thus, a cardiac rhythm management device is described that can monitor electrolyte levels of a patient. The device diagnosis levels based upon a comparative analysis of action potential characteristics recorded from multiple locations. In one embodiment, the electrolyte level is actually quantified.

Although the present disclosure and its advantages have been described in detail, it should be understood that various changes, substitutions and alterations can be made herein without departing from the spirit and scope of the disclosure as defined by the appended claims. For example, although the foregoing description is with reference to an example where the implanted device is a defibrillation/pacer, principles of the invention are applicable to other implantable medical devices as well. In addition, whereas the techniques described are performed by the implanted device, the techniques may alternatively be performed by an external device using IEGM signals or other signals transmitted from the implanted device. For example, a bedside monitor may be configured to receive IEGM signals from the implanted device via "longrange" telemetry, then analyze the signals using the aforementioned techniques and issue any appropriate warnings. Alternatively, the bedside monitor may transmit the IEGM data to a central server or other central processing device, which analyzes data from multiple patients to monitor electrolyte levels within any of those patients. In such an implementation, the central processing device then transmits appropriate warning signals to the bedside monitor of the patient for warning the patient and then additionally transmits appropriate warning signals to the physician associated with the patient or a third party such as emergency medical service (EMS) personnel. A system incorporating bedside monitoring units connected to a centralized external programmer system is described in U.S. Patent No. 6,622,045, issued 09/16/2003 to Snell et al., entitled "System and Method for Remote Programming of Implantable Cardiac Stimulation Devices."

The various functional components of the exemplary systems described herein may be implemented using any appropriate technology including, for example, microprocessors running software programs or application specific integrated circuits (ASICs) executing hard-wired logic operations. The exemplary embodiments of the invention described herein are merely illustrative of the invention and should not be construed as limiting the scope of the invention.

Moreover, the scope of the present application is not intended to be limited to the particular embodiments of the process, machine, manufacture, composition of matter, means, methods and steps described in the specification. As one of ordinary skill in the art will readily appreciate from the present disclosure, processes, machines, manufacture, compositions of matter, means, methods, or steps, presently existing or later to be developed that perform substantially the same function or achieve substantially the same result as the corresponding embodiments described herein may be utilized according to the present disclosure. Accordingly, the appended claims are intended to include within their scope such processes, machines, manufacture, compositions of matter, means, methods, or steps.

**Claims**

1.    A method for diagnosing an electrolyte level with a cardiac rhythm management device, comprising:

   recording intra-cardiac electrograms from a plurality of sites; and
   determining the electrolyte level based upon a comparative analysis of the intra-cardiac electrograms from at least two of the sites.

2.    The method of claim 1, in which the determining further comprises quantifying the electrolyte level using a model including at least one feature of the intra-cardiac electrograms from the at least two sites.

3.    The method of claim 2, further comprising transmitting a warning when the diagnosed electrolyte level exceeds a threshold value.

4.    The method of claim 2, further comprising deriving weighting factors of the model based upon data observed from a plurality of patients.

5.    The method of claim 4, in which the deriving uses normalized features.

6.    The method of claim 4, further comprising calibrating the model to a specific patient based upon a single measured electrolyte level for the specific patient and the corresponding intra-cardiac electrograms from the at least two sites of the specific patient.

7.    The method of claim 6, in which the calibrating further comprises accounting for a plurality of electrolyte level measurements for the specific patient over time and the corresponding intra-cardiac electrograms from the at least two sites of the specific patient.

8.    The method of claim 1, in which the determining the electrolyte level is further based upon a time comparative analysis of the intra-cardiac electrograms from at least two sites.

9.    The method of claim 1, in which the determining further comprises comparing repolarization features of the intra-cardiac electrograms from at least two sites to determine a potassium level or comparing depolarization features of the intra-cardiac electrograms from at least two sites to determine a sodium level or comparing plateau features of the intra-cardiac electrograms from at least two sites to determine a calcium level.

10.    The method of claim 1, further comprising wirelessly transmitting the determined electrolyte level to an external receiver.

11.    A system for diagnosing an electrolyte level of a patient, comprising:

means for recording intra-cardiac electrograms from a plurality of sites; and
means for quantifying the electrolyte level based upon a comparative analysis of the recorded intra-cardiac electrograms from at least two of the sites.

12. The system of claim 11, in which the quantifying means quantifies the electrolyte level based upon intra-cardiac electrograms from the plurality of sites measured at a plurality of times.

13. The system of claim 11, further comprising means for deriving weighting factors of an electrolyte calculating model based upon intra-cardiac electrogram data recorded from the at least two sites in each of a plurality of patients and corresponding measured electrolyte levels in the plurality of patients.

14. The system of claim 13, further comprising means for calibrating the model of a specific patient based upon a single measured electrolyte level for the specific patient and corresponding intra-cardiac electrograms from at least two sites of the specific patient.

15. The system of claim 14, in which the calibrating means further accounts, over time, for a plurality of electrolyte level measurements for the specific patient and the corresponding intra-cardiac electrograms from the at least two sites of the specific patient.

16. The system of claim 11, wherein:

the system comprises a medical device;
the means for recording comprises a first electrode adapted to be implanted at a first cardiac site to facilitate generating a first intra-cardiac electrogram and a second electrode adapted to be implanted at a second cardiac site to facilitate generating a second intra-cardiac electrogram; and
the means for quantifying comprises a processing system that analyzes at least one feature from the first and second intra-cardiac electrograms to quantify the electrolyte level.

17. The system of claim 16, in which the system comprises a cardiac rhythm management device or an external monitor.

EP 2 363 166 A1

FIG. 1

11

FIG. 2

ELECTROLYTE LEVEL MONITORING

FIG. 3

DIAGNOSE ELECTROLYTE LEVEL

FIG. 3A

GENERAL CALIBRATION

```
           ( START )
               |
               v
  +---------------------+
  |  RECORD IEGMs FROM  |  _s40
  |   MULTIPLE SITES OF |
  |   MULTIPLE PATIENTS |
  +---------------------+
               |
               v
  +---------------------+
  |       MEASURE       |  _s41
  |    CORRESPONDING    |
  |  ELECTROLYTE LEVELS |
  +---------------------+
               |
               v
  +---------------------+
  |       DERIVE        |  _s42
  |       MODEL         |
  +---------------------+
               |
               v
           (  END  )
```

FIG. 4

SPECIFIC CALIBRATION

FIG. 5

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 10 25 0952

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/083092 A1 (RIPPO ANTHONY J [US] ET AL) 12 April 2007 (2007-04-12) * paragraph [0033] - paragraph [0044] * ----- | 11-17 | INV. A61N1/368 ADD. A61N1/372 |
| A | US 2008/188761 A1 (COUDERC JEAN-PHILIPPE [US]) 7 August 2008 (2008-08-07) * paragraph [0028] - paragraph [0079] * ----- | 11-17 | |
| A | US 6 572 542 B1 (HOUBEN RICHARD [NL] ET AL) 3 June 2003 (2003-06-03) * column 7, line 35 - column 23, line 64 * ----- | 11-17 | |

TECHNICAL FIELDS
SEARCHED      (IPC)

A61N

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 28 July 2011 | Sopelana Martínez, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04E07)

Claim(s) completely searchable:
         11-17

Claim(s) not searched:
         1-10

Reason for the limitation of the search:

The search has been restricted to the subject-matter indicated by the
applicant in his letter of 26.08.2010 filed in reply to the invitation
pursuant to Rule 63(1) EPC. Thus, the search report has been drawn up on
the basis of claims 11 - 17.

**ANNEX TO THE EUROPEAN SEARCH REPORT
ON EUROPEAN PATENT APPLICATION NO.**

EP 10 25 0952

This annex lists the patent family members relating to the patent documents cited in  the above-mentioned European search report.
The members are as contained in the European Patent Office EDP file on
The European Patent Office is in no way liable for these particulars which are merely given for the purpose of information.

28-07-2011

| Patent document cited in search report | Publication date | Patent family member(s) | Publication date |
|---|---|---|---|
| US 2007083092 A1 | 12-04-2007 | US 2007083095 A1 | 12-04-2007 |
| US 2008188761 A1 | 07-08-2008 | NONE | |
| US 6572542 B1 | 03-06-2003 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6622045 B, Snell